(19)

Europäisches
Patentamt
European
Patent Office
Office européen
des brevets

(11) **EP 3 207 133 B1**

(12) **EUROPEAN PATENT SPECIFICATION**

(45) Date of publication and mention
of the grant of the patent:
**05.05.2021 Bulletin 2021/18**

(21) Application number: **15797426.2**

(22) Date of filing: **16.10.2015**

(51) Int Cl.:
***C12N 15/09*** (2006.01)

(86) International application number:
**PCT/IB2015/057952**

(87) International publication number:
**WO 2016/059604 (21.04.2016 Gazette 2016/16)**

(54) **CROSS-PLATFORM TRANSFORMATION OF GENE EXPRESSION DATA**

PLATTFORMÜBERGREIFENDE TRANSFORMATION VON GENEXPRESSIONSDATEN

TRANSFORMATION ENTRE PLATEFORMES DE DONNÉES D'EXPRESSION DE GÉNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.10.2014 US 201462065367 P**

(43) Date of publication of application:
**23.08.2017 Bulletin 2017/34**

(73) Proprietor: **Koninklijke Philips N.V.**
**5656 AG Eindhoven (NL)**

(72) Inventors:
• **CHEUNG, Yee Him**
**NL-5656 AE Eindhoven (NL)**
• **VERHAEGH, Wilhelmus Franciscus Johannes**
**NL-5656 AE Eindhoven (NL)**
• **DIMITROVA, Nevenka**
**NL-5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(56) References cited:
• BAUER MICHAEL A ET AL: "Leveraging the new with the old: providing a framework for the integration of historic microarray studies with next generation sequencing", BMC BIOINFORMATICS, vol. 15, no. Suppl. 11, 1 October 2014 (2014-10-01), XP002753058,
• SHWETA S CHAVAN ET AL: "Towards the integration, annotation and association of historical microarray experiments with RNA-seq", BMC BIOINFORMATICS, BIOMED CENTRAL, LONDON, GB, vol. 14, no. Suppl 14, S4, 9 October 2013 (2013-10-09), pages 1-11, XP021163013, ISSN: 1471-2105, DOI: 10.1186/1471-2105-14-S14-S4
• ZHAO SHANRONG ET AL: "Comparison of RNA-Seq and Microarray in Transcriptome Profiling of Activated T Cells", PLOS ONE, vol. 9, no. 1, January 2014 (2014-01), XP002753059,
• MANTIONE KIRK J ET AL: "Comparing bioinformatic gene expression profiling methods: microarray and RNA-Seq.", MEDICAL SCIENCE MONITOR BASIC RESEARCH 2014, vol. 20, 27 July 2014 (2014-07-27), pages 138-141, XP002753060, ISSN: 2325-4416, DOI: 10.12659/MSMBR.892101

**Description**

CROSS-REFERENCE TO RELATED APPLICATIONS

The present application is related to co-pending United States provisional application no. 62/065,367, filed on October 17, 2014.

TECHNICAL FIELD

Embodiments of the present invention relate generally to the use of gene expression data, and in particular to the use of gene expression data across different profiling platforms.

BACKGROUND

The dynamic ranges of gene expressions can vary considerably depending on the choice of profiling platform. As a result, prognostic gene signatures are usually platform-specific. In general, expression data generated by heterogeneous platforms cannot be directly combined for computational analysis, thus limiting the use of legacy data and hindering the adoption of new profiling technologies. More specifically, in can be difficult to transfer the knowledge and insight gained from the tremendous amount of legacy microarray studies onto new platforms such as Next Generation Sequencing (NGS) systems.

A number of methods have been proposed to handle cross-platform compatibility of expression data. One approach involves mapping probes/reads to common genomic targets, then calling platform-level expression (RMA for microarray and RPKM for RNA-Seq) for each target, and finally applying quantile normalization, assuming that the expression distributions across platforms only differ by a sample-specific scaling factor. Another approach involves the application of gene-by-gene factor analysis to obtain a unified expression measure from multiple platforms using expectation-maximization (EM) algorithms. Yet another approach uses a system of functional measurement error models to model gene expression measurements and calibrate platforms using the allegedly more reliable but low-throughput qRT-PCR expressions for a subset of the genes. Like factor analysis, however, the models may only hold for an expression range that fits all three platforms and genes with extreme expressions are excluded. Still another approach involves counting the number of reads overlapping with probe regions in RNA-Seq data, estimating probe-region expressions using an empirical Bayesian approach, and subsequently applying a modified RMA algorithm (i.e., without the background correction step) on the probe-region expressions to obtain gene-level expressions. This method, however, involves more complicated computations on the mapped reads and is rigid in terms of the choice of platforms, i.e. RNA-Seq for input and RMA for output.

BAUER MICHAEL A ET AL, "Leveraging the new with the old: providing a framework for the integration of historic microarray studies with next generation sequencing", BMC BIOINFORMATICS, (20141001), vol. 15, no. Suppl. 11, describes features of a suite of software tools for integrating, exploring, discovering and validating profiling data from different modalities with one another. SHWETA S CHAVAN ET AL, "Towards the integration, annotation and association of historical microarray experiments with RNA-seq", BMC BIOINFORMATICS, BIOMED CENTRAL, LONDON, GB, (20131009), vol. 14, no. Suppl 14, doi:10.1186/1471-2105-14-S14-S4, ISSN 1471-2105, pages 1 - 11, describes software approaches for integrating, cross-referencing and associating microarray and RNA-seq gene expression datasets.

Given the limitations of these prior approaches, it would be desirable to have a generalized approach that supports the transformations of measurements from one gene expression platform to another.

SUMMARY

This summary is provided to introduce a selection of concepts in a simplified form that are further described below in the Detailed Description section. This summary is not intended to identify key features or essential features of the claimed subject matter, nor is it intended to be used as an aid in determining the scope of the claimed subject matter.

Aspects of the present invention relate to a data-driven generalized regression-based framework that supports the transformation of measurements, applicable but not limited to gene expressions, from one platform to another over a wide dynamic range, with selected summary statistics / feature values as predictors for the model parameters. The framework consists of primary model training and transformation, and additional levels of categorical regression and transformation processes.

Embodiments of the present invention eliminate the unnecessary re-profiling of samples for the sake of combined analysis, solve the backward compatibility problem, and facilitate the adoption of new profiling technologies by allowing legacy data to be readily transformed for use with data from newer platforms. Furthermore, platform-specific gene signatures can be extended for use on the expression data from multiple platforms, either by transforming the input data to the primary platform or by adapting the parameters of a signature to the alternative platforms.

According to one aspect of the disclosure, embodiments of the present invention relate to a method for facilitating direct combination, for computational analysis, of gene expression data generated by heterogeneous platforms according to claim 1.

In some embodiments, constructing the model further includes generating at least one set of expression data using a profiling platform, the at least one set of expression data being at least one of the first and second sets of expression data.

In some embodiments, the method includes transforming the sample expression data using the constructed primary model. In some embodiments, the method includes constructing a categorical model by regression analysis from at least one of: (a) at least some of the transformed sample expression data and (b) at least some of the common expressions. In some embodiments, at least one of the: (a) selection of at least some of the transformed sample expression data and (b) selection of at least some of the common expressions, is based on phenotypic data or any factor known to introduce cross-platform bias. In some embodiments, the method includes iterating this process using the categorical model constructed from the transformed sample expression data to transform the transformed sample expression data and constructing another categorical model therefrom. In some embodiments, the method includes transforming a set of expression data from the first profiling platform to the second profiling platform by applying the constructed categorical models in the order of their construction.

In some embodiments, the first profiling platform or the second profiling platform is selected from the group consisting of but not restricted to Agilent Gene Expression Microarrays, Affymetrix Gene Profiling Array cGMP U133 P2 / Human Genome U133 Plus 2.0 /U133A 2.0, Illumina Genome Analyzer/MiSeq/NextSeq/HiSeq, NanoString nCounter SPRINT/MAX/FLEX, and Oxford Nanopore MinION/PromethION/GridION. In some embodiments, the at least one common expression is identified by at least one of matching genomic positions, matching exons, matching isoforms, and matching transcripts. In some embodiments, the at least one candidate feature is selected from the group consisting of mean transcript expression, mean normalized probe intensity, number of detected genes, total number of reads per sample, average numbers of reads per exon/gene/isoform, read coverage, and any other appropriate statistics of each sample. In some embodiments, each of the models is selected from the group consisting of a linear model, a logarithmic model, a piecewise linear model, and any other appropriate regression model.

According to another aspect of the disclosure, embodiments of the present invention relate to an apparatus for facilitating direct combination, for computational analysis, of gene expression data generated by heterogeneous platforms according to claim 11.

In some embodiments, the interface is configured to receive at least one set of expression data from a profiling platform, the at least one set of expression data being at least one of the first and second sets of expression data.

In some embodiments, the apparatus further includes computer executable instructions operative on said processor for transforming the sample expression data using the constructed primary model. In some embodiments, the apparatus further includes computer executable instructions operative on said processor for constructing a categorical model by regression analysis from at least one of: (a) at least some of the transformed sample expression data and (b) at least some of the common expressions. In some embodiments, at least one of the: (a) selection of at least some of the transformed sample expression data and (b) selection of at least some of the common expressions, is based on phenotypic data or any other factor known to introduce cross-platform bias. In some embodiments, the apparatus further includes computer executable instructions operative on said process for iterating this process using the categorical model constructed from the transformed sample expression data to transform the transformed sample expression data and construct another categorical model therefrom. In some embodiments, the apparatus further includes computer executable instructions operative on said processor for transforming a set of expression data from the first profiling platform to the second profiling platform by applying the constructed categorical models in the order of their construction.

In some embodiments, the first profiling platform or the second profiling platform is selected from the group consisting of but not restricted to Agilent Gene Expression Microarrays, Affymetrix Gene Profiling Array cGMP U133 P2 / Human Genome U133 Plus 2.0 /U133A 2.0, Illumina Genome Analyzer/MiSeq/NextSeq/HiSeq, NanoString nCounter SPRINT/MAX/FLEX, and Oxford Nanopore MinION/PromethION/GridION.

In some embodiments, the computer executable instructions for identifying at least one common expression include computer executable instructions for identifying at least one common expression by at least one of matching genomic positions, matching exomes, matching isoforms, and matching transcripts. In some embodiments, the at least one candidate feature is selected from the group consisting of mean transcript expression, mean normalized probe intensity, number of detected genes, number of reads per sample, average number of reads per exon/gene/isofrom, read coverage, and any other appropriate statistics of each sample. In some embodiments, each of the models is selected from the group consisting of a logarithmic model, a linear model, a piecewise linear model, and a regression model.

These and other features and advantages, which characterize the present non-limiting embodiments, will be apparent from a reading of the following detailed description and a review of the associated drawings. It is to be understood that both the foregoing general description and the following detailed description are explanatory only and are not restrictive of the non-limiting embodiments as claimed.

BRIEF DESCRIPTION OF DRAWINGS

The accompanying drawings are not intended to be drawn to scale. In the drawings, each identical or nearly identical component that is illustrated in various figures may be represented by a like numeral. For purposes of clarity, not every component may be labeled in every drawing. Various embodiments of the invention will now be described, by way of example, with reference to the accompanying drawings, in which:

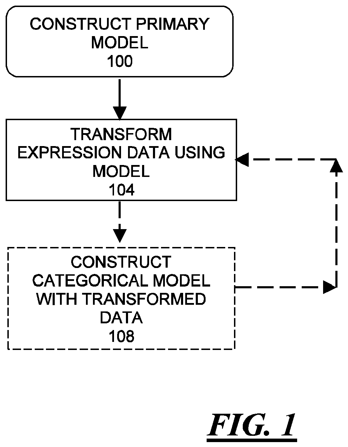

FIG. 1 is a flowchart of a process for transforming gene expression data from a first platform to a second platform;

FIG. 1 is a flowchart of a model training process according to one embodiment of the present invention;

FIG. 2 is a flowchart of a primary model construction process according to one embodiment of the present invention;

FIG. 3 is a flowchart of a process for transforming sample data using the primary model according to one embodiment of the present invention;

FIG. 4 is a flowchart of a categorical regression process in accord with one embodiment of the present invention;

FIG. 5 is a flowchart of a process for transforming additional data using models in accord with one embodiment of the present invention;

FIG. 6 is an illustration of a stack of transformation models developed and applied in accord with one embodiment of the present invention;

FIG. 7 presents the linear models between mean expression level and regression parameters developed in one embodiment of the present invention;

FIG. 8 shows the predicted piecewise-linear model for each sample;

FIG. 9 shows the relationship between the transformed expression and the original microarray expression after (A) first-level sample-wise transformation, and (B) second-level gene-wise transformation respectively; and

FIG. 10 presents a block diagram of an embodiment of an apparatus for cross-platform transformation of gene expression data in accord with the present invention.

DETAILED DESCRIPTION

Cross-platform compatibility of gene expression data is a crucial and active topic of research. It can be inefficient to manage and analyze sample data originated from a mixture of platforms. For instance, the Cancer Genome Atlas (TCGA) currently has five different platforms for RNA expression: Agilent G4502A, Affymetrix HT-HG_U133A, HG-U133_Plus_2, Illumina GA and Illumina HiSeq 2000, thus making it difficult to leverage the full potential of the data through a combined analysis. Furthermore, the dynamic ranges of gene expressions can vary considerably depending on the choice of profiling platform.

With the huge amount of legacy data generated throughout the years based on former technologies, the diversity of existing platforms, and the emergence of new ones, it can be advantageous to provide compatibility of data across various platforms. Breaking platform barriers means saving the cost of re-profiling of samples in order to perform combined analysis. It can also solve the backward compatibility problem and facilitate the adoption of new profiling technologies by allowing legacy data to be readily transformed for use with data from newer platforms. Specifically, tremendous resources have been spent on microarray studies, and it is desirable to transfer the knowledge and insights from these studies onto new platforms such as next-generation sequencing (NGS) technologies.

Embodiments of the present invention facilitate cross-platform compatibility of gene expression data using models that transform expression data from one platform to another. These embodiments can also, in the clinical research setting, be applied across different cohorts available to a clinical researcher in order to evaluate many signatures on a new cohort, either by transforming the input data to the primary platform or by adapting the parameters of a signature to the alternative platforms.

With reference to FIG. 1, one embodiment of a method in accord with the present invention for transforming gene expression data begins with the construction of a primary model (Step 100) for directly transforming expression data from a first profiling platform to a second profiling platform without an intermediate transformation. The primary model may then be used to transform expression data from the first platform to the second platform (Step 104).

However, in some embodiments, the model construction process may include additional levels of iteration. In these embodiments, an additional model, e.g., a categorical regression model, may be constructed from transformed expression data (Step 108). This additional model may be used, in turn, to transform additional expression data (Step 104). This process may be iterated through additional rounds of categorical model construction (Step 108) and the application of those categorical models to transform expression data (Step 104), which may then be used to construct additional categorical models (Step 108) and so on. When multiple categorical models are constructed and subsequently used to transform expression data, the models are applied in the order of their construction - i.e., the first model constructed is the first model used to transform data, the second model constructed is used to transform the data transformed by the first model, and so on.

Construction of Primary Model

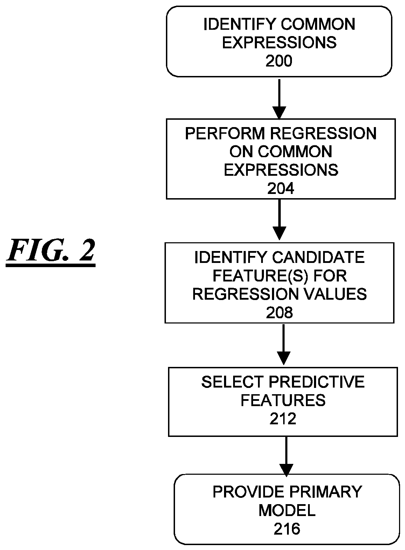

As discussed above, embodiments of the present invention typically construct a primary model for transforming data sets between platforms (Step 100). With reference to FIG. 2, the model training process begins with the identification of at least one common gene target (Step 200) between the expression data for Platform X (i.e., $x_i = \{x_{gj}\}_{i,j} = 1 \ldots K$, where $g_j$ denotes the $K$ gene targets for Platform $X$) and the expression data for Platform Y ($y_i = \{y_{gj}\}_{i,j} = 1 \ldots L$, with $L$ gene targets for Platform Y).

If there is no direct mapping between the two sets of targets, the targets could be mapped from set to another by their genomic positions. For example, if the source data are RNA-Seq exon expressions and the destination data are microarray gene expressions, then exons that overlap with the microarray probe-sets can be identified and summarized into gene expressions before applying regression.

With $\{S_i\}_i = 1 \ldots N$ referring to the $N$ training samples available to construct the model for transforming gene expression data from Platform $X$ to Platform $Y$, for each sample $S_i$, regression is performed between $x_i$, and $y_i$ using expressions that are detected on both platforms (Step 204).

The target model for the regression process is assumed *a priori* to be defined by $M$ parameters. Depending on the observed relationship between the training data from the source and destination platforms, any regression model can be chosen that results in the least error, such as non-linear, logarithmic, LOESS (local regression) or errors-in-variables (EiV) models. In addition, an optimization function can be applied to choose a model with the least error. This choice may be the decision of a human operator, or it may be the outcome of an automated or a semi-automated process. With an appropriate model selected, the output of the regression process is $N$ sets of parameters $r_i = \{r_k\}_{i,k} = 1 \ldots M$.

Given the regression parameters $r_i$ for each sample $S_i$, candidate features $f$ are selected from the data generated by Platform $X$ that can be good predictors for the regression parameters (Step 208). For example, if Platform $X$ is a microarray platform, the candidates $f$ may include mean expression, mean normalized probe intensity, etc. If Platform $X$ is an RNA-Seq platform, the candidates $f$ may include mean expression, number of detected genes, total number of reads, read coverage, etc. As with the choice of regression model, the identification of candidate features $f$ may be performed by a human operator or by an automated or semi-automated process.

It is not necessary for the predictive features to be extracted only from the source data. Sometimes features from the target data may have good performance in predicting the regression parameters. In some embodiments, such target platform features may also be included in the model and, e.g., assigned with the mean value of the feature in the training data for the transformation process.

Having identified possible candidate features $f$ from Platform $X$ (Step 208), those features $f_k$ that actually predict the regression parameters $r_i$, need to be identified from the set of possible candidate features $f$ (Step 212). In one embodiment, the predictive features may be identified by means of, e.g., stepwise regression or other automated, manual, or semi-automated methods. If the goal is to select a single predictive feature for each parameter instead of a subset, the feature with the highest correlation with the parameter can be selected.

The output of the model construction process consists of the identified predictive features $f_k$ and their corresponding models $\gamma_k$ for the prediction of the regression model parameters $r_i$ for each sample S, (Step 216).

In some embodiments, the expression data for a particular platform (e.g,. $x_i$ for Platform $X$, $y_i$ for Platform $Y$, etc.) with appropriate normalization is generated for the training samples $\{S_i\}$ (not shown) prior to the identification of the common expressions (Step 200).

Primary Model Transformation

Once the primary model has been created, it may be used to transform subsequent samples from Platform $X$ to Platform $Y$. Assume for the following discussion that there exists data generated on Platform $X$ for a new sample $P_n$.

That data includes the expression profile $z_n$, and sets of predictive feature values $\{v_k\}_n$ that correspond to the predictive features $\{f_k\}_k$= 1, ...,M discussed above in connection with FIG. 2.

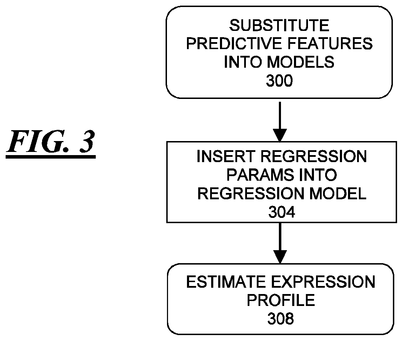

With reference to FIG. 3, the transformation of the new data associated with $P_n$ begins with the substitution of the predictive feature values $\{v_k\}_n$ into their respective models $\{\gamma_k\}$, resulting in the regression model parameters $r_n$ for $S_i$ (Step 300).

The predicted regression model parameters $r_n$ can be applied to a pre-defined regression model (Step 304), enabling the estimation of the expression profile as $\widehat{z}_n^{(0)} = \{\hat{z}_{g_j}\}_{n,j=1,...,K}^{(0)}$ for sample $P_n$ (Step 308).

Categorical Model Construction and Transformation

In some embodiments, the primary model may suffice to transform expression data between profiling platforms. As discussed above, in other embodiments additional levels of categorical modeling and transformation may be employed to convert data between platforms.

In particular, if there are one or more factors that introduce additional cross-platform discrepancies, then additional levels of regression related to the factors can be performed on the data, with the transformed data from the previous level of regression serving as the input to the next level of regression.

For example, assume the existence of one factor of well-defined categories $c_l = \{c_m\}_l$, m = 1, ..., $O_l$, where l = 1 is the factor index, that introduces additional cross-platform discrepancy on top of the sample-wise transformation. In general, categories can be defined based on gene models as well as natural groupings across samples (e.g. normal vs. tumor, different clinical staging, different subtypes, different outcomes, etc.). For example, gene symbol can be a useful factor for the transformation of gene expressions between microarray and RNA-Seq platforms due to the non-uniform distributions of microarray probe-sets along each gene and alternative splicings. If genes within a category show strong correlation with each other, multi-variate regression can be applied so that each transformed expression value is based on the group of coexpressed genes.

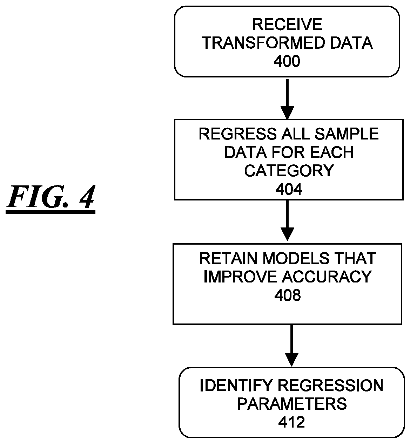

With reference to FIG. 4, the categorical modeling process begins with the receipt of transformed data, either from the primary model or from a prior level of categorical modeling (Step 400). Next, regression stratified by the categories is performed on all the received transformed values (Step 404). For each category, those models that significantly improve accuracy (e.g., root-mean-square error) are identified and retained (Step 408). The identification of those models yields additional sets of regression parameters $\{q_m\}_l$ for each of the categories that can enhance the accuracy of the transformed data (Step 412).

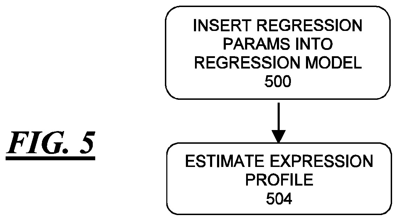

With reference to FIG. 5, the further transformation of the transformed data associated with $P_n$ begins with the substitution of the regression parameters $\{q_m\}_l$ into their respective regression models (Step 500). These models can then be used to estimate the expression profile as $\widehat{z}_n^{(l)} = \{\hat{z}_{g_j}\}_{n,j=1,...,K}^{(l)}$ for sample $P_n$ at the lth level of transformation (Step 504).

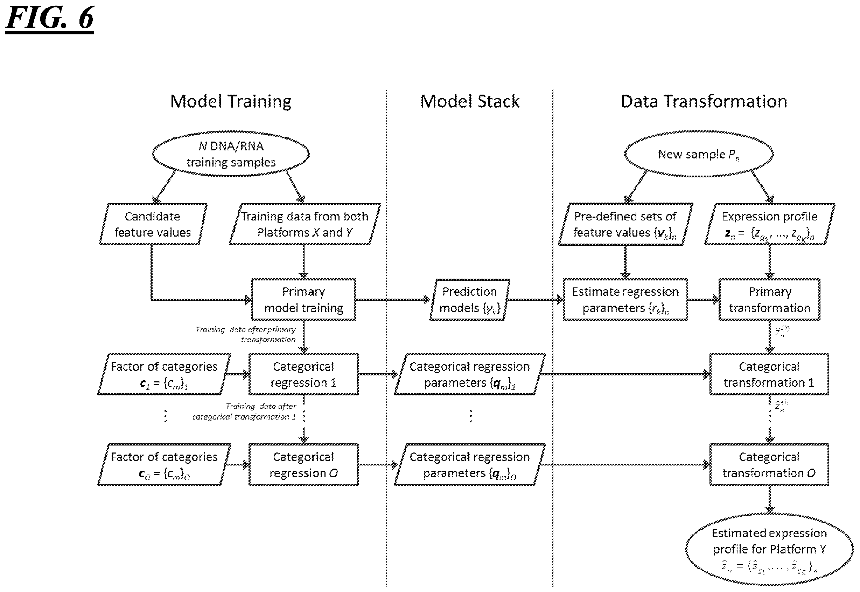

This process of categorical modeling and transformation of the training data shown in FIGS. 4 and 5 can be repeated for multiple independent factors to enhance transformation accuracy. The result is a "stack" of order-dependent regression models, with the primary sample-wise regression at the bottom and additional layers for each categorical factor on top of each other. FIG. 6 presents one example of such an arrangement for O independent factors. It should be noted that, when applying a stack of transformation models, the order of the models applied must be the same as the order of construction followed in the model construction process.

Exemplary Embodiments

According to one embodiment, there is provided a system and method for the transformation of gene expression data (in $\log_2$ scale) from Affymetrix GeneChip HT Human Genome U133 Array Plat Set (RMA) to Illumina HiSeq 1000 RNA-Seq (RSEM) using 545 TCGA samples that have data generated on each of the respective platforms. Some sample-wise statistics are summarized for the two platforms in Table 1. The mean correlation per sample is 0.713 and higher expressions show stronger correlation in general.

| Across Samples | Affymetrix Microarray (log2 RMA) | | Illumina RNA-Seq (log2 RSEM) | | Correlation |
|---|---|---|---|---|---|
| | Mean | Variance | Mean | Variance | |
| Min. | 5.143 | 2.939 | 7.307 | 7.211 | 0.425 |
| Max. | 6.687 | 4.862 | 8.232 | 9.699 | 0.782 |
| Mean | 5.804 | 4.116 | 7.795 | 8.363 | 0.713 |
| Variance | 0.201 | 0.139 | 0.017 | 0.178 | 0.001 |

Table 1 – Summary statistics of TCGA samples with expression data generated on both Affymetrix microarray and Illumina RNA-Seq platforms

By generating scatterplots of RNA-Seq vs. microarray expressions for each sample, it can be seen that their relationship can be suitably approximated by a piecewise linear model. In an exemplary implementation using the R programming language, the 'lm' function for linear regression and the 'segmented' function of the 'segmented' package are applied for breakpoint ($x_b$) estimation. This resulted in four regression parameters $\{m_1, c_1, m_2, c_2\}$ for the linear models before and after the estimated breakpoint: $y_1 = m_1x_1 + c_1$ for $x \leq x_b$ and $y_2 = m_2x_2 + c_2$ for $x > x_b$. Summary statistics of the regressed piecewise linear models are summarized in Table 2 below.

| Across Samples | Before Breakpoint ($x \leq x_b$) | | After Breakpoint ($x > x_b$) | | Breakpoint ($x_b$) |
|---|---|---|---|---|---|
| | Slope 1 ($m_1$) | Intercept 1 ($c_1$) | Slope 2 ($m_2$) | Intercept 2 ($c_2$) | |
| Min. | 1.704 | -14.570 | 0.338 | 3.378 | 4.221 |
| Max. | 4.892 | -0.866 | 0.876 | 7.044 | 5.747 |
| Mean | 3.849 | -9.810 | 0.641 | 5.421 | 4.778 |
| Variance | 0.154 | 2.264 | 0.004 | 0.344 | 0.134 |

Table 2 – Summary statistics of the regressed piecewise linear models.

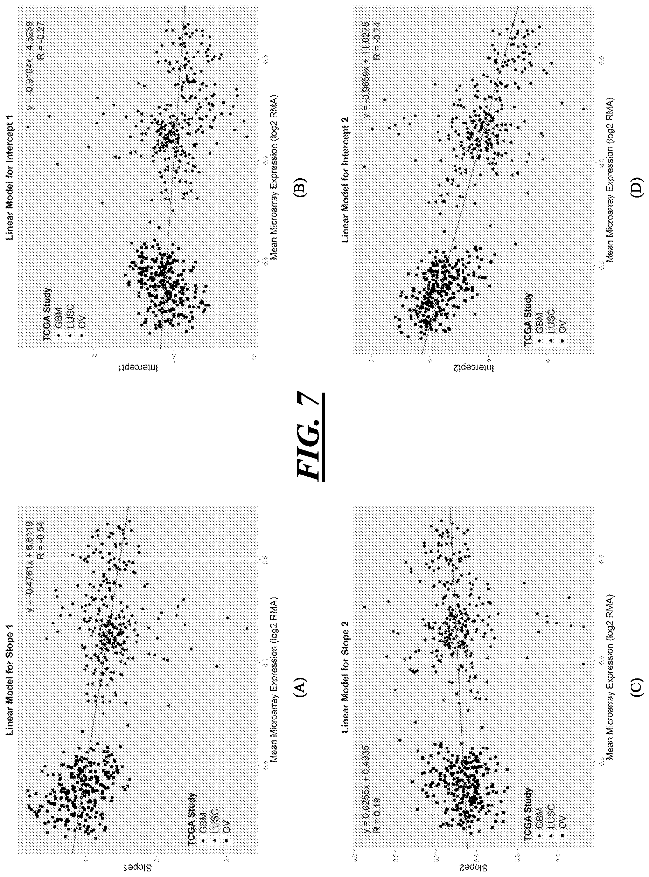

Next, a small set of candidate features for predicting the four regression model parameters are generated, and it can be determined that mean expression level was a plausible single linear predictor, with moderately strong correlations of $R = -0.55$ with $m_1$ and $R = 0.74$ with $c_2$, despite lesser correlations of $R = -0.27$ with $c_1$ and $R = 0.19$ with $m_2$, which has a small variance of 0.04. The linear models between mean expression level and the four regression parameters are shown in FIGS. 7(A) Slope 1, 7(B) Intercept 1, 7(C) Slope 2 and 7(D) Intercept 2.

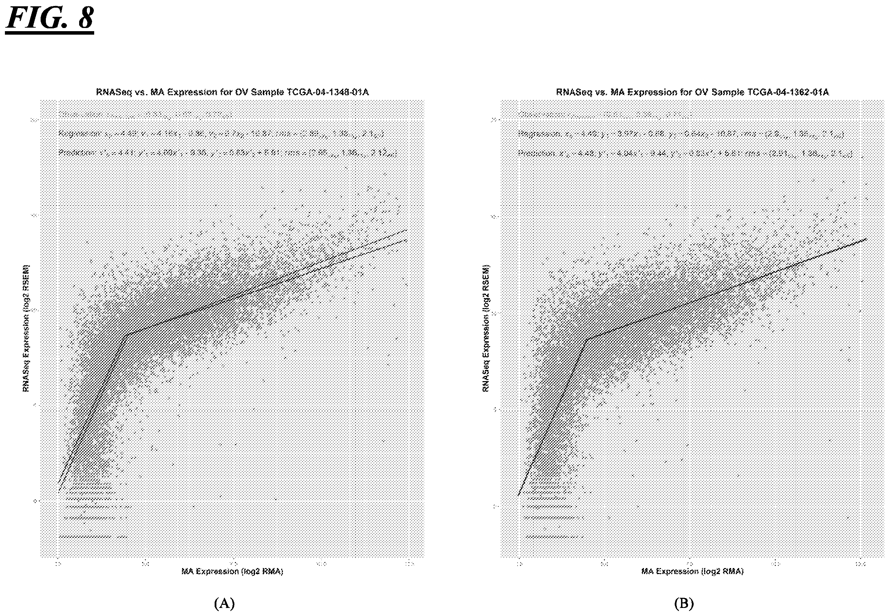

Using mean expression level as a predictor, the piecewise-linear model for each sample can be predicted. FIGS. 8(A) and 8(B) show the piecewise-linear models based on direct regression and a prediction method for two samples, i.e., cross-platform scatterplot with the regressed and predicted models for two TCGA samples.

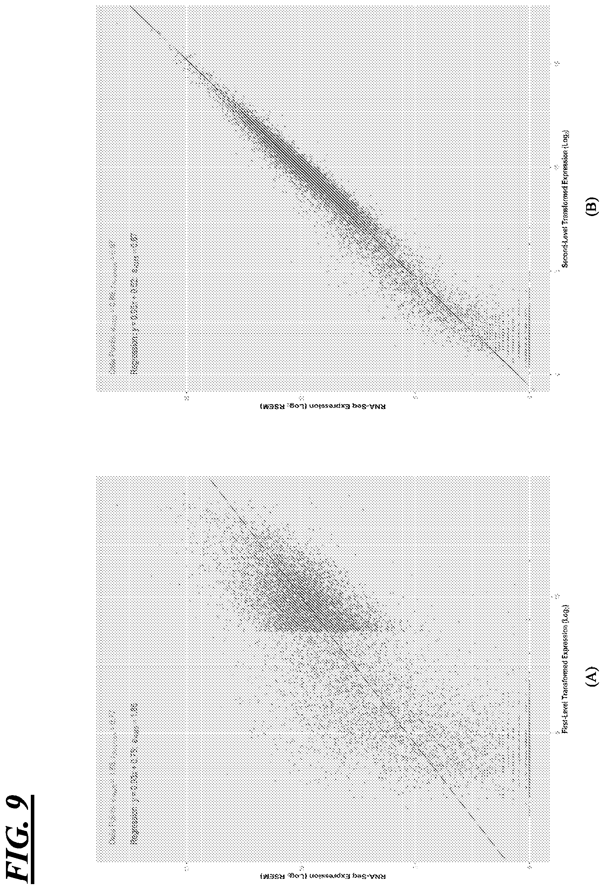

As illustrated, for moderate-to-high microarray expressions, the predicted RNA-Seq expressions have a root-mean-square error $e_{rms} = 1.4$, which is very close to that of 1.39 based on the estimated values obtained by direct regression. To further improve the accuracy, an additional level of regression and transformation can be applied on the primarily transformed values stratified by genes across all samples using the categorical approach as described above. FIGS. 9(A) and 9(B) show the relationship between the transformed expression and the original microarray expression after respectively the first-level sample-wise transformation and the second-level gene-wise transformation for one sample.

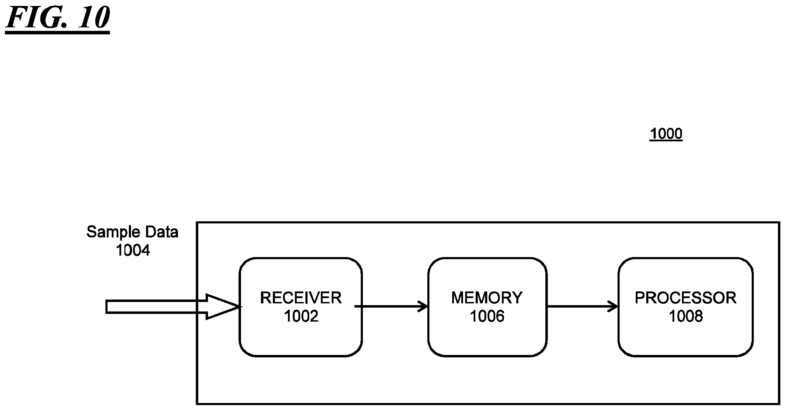

Referring to FIG. 10, a schematic representation 1000 of a system for transforming gene expression data in accordance with one embodiment of the invention is illustrated. The system 1000 includes a receiver 1002 for receiving sample expression data 1004, a memory 1006 configured to store the received sample expression data 1004 and a processor 1008.

Processor 1008 is configured as discussed above to build primary and categorical models for transforming gene expression data from a first profiling platform to a second profiling platform such that the overall distribution of the transformed data resembles that of the second platform.

Applications

Embodiments of the present invention may be extended to compute unified expressions from data measured by multiple platforms. For instance, all data may be transformed to one specific platform using, e.g., an EiV regression model, and then for each target combine the transformed values with weighted averaging using weights that are inversely proportional to the estimated noise variances of the respective source platforms.

While the above embodiments of present invention are described with respect to measurements performed on genomics platforms, the same process and procedures can be applied to physiology modelling, imaging, personal continuous health data and others.

Although above embodiments of the present invention are described with respect to gene expression data, the process and procedures described herein are applicable to solving the compatibility problem across different platforms or analytical pipelines of any numerical readings. For example, methylation levels, protein expressions or even sensor measurements, with structural discrepancies due to the inherent differences of the underlying systems.

Equivalents, definitions, etc.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present invention.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, e.g., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, e.g., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (e.g. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," and the like are to be understood to be open-ended, e.g., to mean including but not limited to.

Only the transitional phrases "consisting of' and "consisting essentially of' shall be closed or semi-closed transitional phrases, respectively

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

It should also be understood that, unless clearly indicated to the contrary, in any methods claimed herein that include more than one step or act, the order of the steps or acts of the method is not necessarily limited to the order in which the steps or acts of the method are recited.

## Claims

1. A computer implemented method for facilitating direct combination of gene expression data generated by heterogeneous platforms, the method comprising transforming gene expression data, the method comprising:

   constructing a primary model for transforming gene expression data from a first profiling platform to a second profiling platform utilizing sample expression data, wherein constructing the primary model comprises:
   identifying at least one common expression between a first set of nucleic acid expression data derived using a first profiling platform and a second set of nucleic acid expression data derived using a second profiling platform, each common expression associated with a sample present in both the first set and second set;
   performing regression analysis on the at least one common expression, resulting in one set of regression parameters for each sample;
   selecting at least one candidate feature from the first profiling platform that predicts the at least one set of regression parameters; and
   identifying a primary model associated with each of the at least one selected candidate features ,the primary model being configured for sample-wise data transformation to transform gene expression data from the first profiling platform into transformed data having a distribution which resembles that of the second profiling platform.

2. The method of claim 1, further comprising generating at least one set of expression data using a profiling platform, the at least one set of expression data being at least one of the first and second sets of expression data.

3. The method of claim 1, further comprising:

   transforming the sample expression data using the constructed primary model; and
   constructing a categorical model by regression analysis from at least one of: (a) at least some of the transformed sample expression data and (b) at least some of the common expressions.

4. The method of claim 3, wherein at least one of the: (a) selection of at least some of the transformed sample expression data and (b) selection of at least some of the common expressions, is based on phenotypic data or any factor known to introduce cross-platform bias.

5. The method of claim 3, further comprising iterating claim 4 using the categorical model constructed from the transformed sample expression data to transform the transformed sample expression data and constructing another categorical model therefrom.

6. The method of claim 5, further comprising transforming a set of expression data from the first profiling platform to the second profiling platform by applying the constructed categorical models in the order of their construction.

7. The method of claim 1, wherein the first profiling platform or the second profiling platform is selected from the group consisting of Agilent Gene Expression Microarrays, Affymetrix Gene Profiling Array cGMP U133 P2 / Human Genome U133 Plus 2.0 /U133A 2.0, Illumina Genome Analyzer/MiSeq/NextSeq/HiSeq, NanoString nCounter SPRINT/MAX/FLEX, and Oxford Nanopore MinION/PromethION/GridION.

8. The method of claim 1, wherein the at least one common expression is identified by at least one of matching genomic positions, matching exons, matching isoforms, and matching transcripts.

9. The method of claim 1, wherein the at least one candidate feature is selected from the group consisting of mean transcript expression, mean normalized probe intensity, number of detected genes, number of reads per sample, average number of reads per exon/gene/isoform, read coverage, and a sample statistic.

10. The method of claim 5, wherein each of the models is selected from the group consisting of a linear model, a logarithmic model, a piecewise linear model, and a regression model.

11. An apparatus for facilitating direct combination, for computational analysis, of gene expression data generated by heterogeneous platforms by transforming gene expression data, the apparatus comprising:

a processor;
an interface; and
computer executable instructions operative on said processor for:

constructing a primary model for transforming gene expression data from a first profiling platform to a second profiling platform utilizing sample expression data such that the overall distribution of the transformed data resembles that of the second platform,
wherein the computer executable instructions for constructing the primary model comprise computing executable instructions for:

identifying at least one common expression between a first set of nucleic acid expression data derived using a first profiling platform and a second set of nucleic acid expression data derived using a second profiling platform, each common expression associated with a sample present in both the first set and second set;
performing regression analysis on the at least one common expression, resulting in one set of regression parameters for each sample;
selecting at least one candidate feature from the first profiling platform that predicts the at least one set of regression parameters; and
identifying a primary model associated with each of the at least one selected candidate features the primary model being configured for sample-wise data transformation to transform gene expression data from the first profiling platform into transformed data having a distribution which resembles that of the second profiling platform.

12. The apparatus of claim 11, wherein the interface is configured to receive at least one set of expression data from a profiling platform, the at least one set of expression data being at least one of the first and second sets of expression data.

13. The apparatus of claim 11, further comprising computer executable instructions operative on said processor for:

transforming the sample expression data using the constructed primary model; and
constructing a categorical model by regression analysis from at least one of: (a) at least some of the transformed sample expression data and (b) at least some of the common expressions.

14. The apparatus of claim 13, wherein at least one of the: (a) selection of at least some of the transformed sample expression data and (b) selection of at least some of the common expressions, is based on phenotypic data or any factor known to introduce cross-platform bias.

15. The apparatus of claim 13, further comprising computer executable instructions operative on said processor for iterating claim 13 using the categorical model constructed from the transformed sample expression data to transform the transformed sample expression data and construct another categorical model therefrom.

16. The apparatus of claim 15, further comprising computer executable instructions operative on said processor for transforming a set of expression data from the first profiling platform to the second profiling platform by applying the constructed categorical models in the order of their construction.

17. The apparatus of claim 11, wherein the first profiling platform or the second profiling platform is selected from the group consisting of Agilent Gene Expression Microarrays, Affymetrix Gene Profiling Array cGMP U133 P2 / Human Genome U133 Plus 2.0 /U133A 2.0, Illumina Genome Analyzer/MiSeq/NextSeq/HiSeq, NanoString nCounter SPRINT/MAX/FLEX, and Oxford Nanopore MinION/PromethION/GridION.

18. The apparatus of claim 11, wherein the computer executable instructions for identifying at least one common expression comprise computer executable instructions for identifying at least one common expression by at least one of matching genomic positions, matching exons, matching isoforms, and matching transcripts.

19. The apparatus of claim 11, wherein the at least one candidate feature is selected from the group consisting of mean transcript expression, mean normalized probe intensity, number of detected genes, number of reads per sample, average number of reads per exon/gene/isoform, read coverage, and a sample statistic.

20. The apparatus of claim 15, wherein each of the models is selected from the group consisting of a logarithmic model, a linear model, a piecewise linear model, and a regression model.

**Patentansprüche**

1. Eine auf einem Computer umzusetzende Methode für das direkte Kombinieren von Genexpressionsdaten, die von heterogenen Plattformen erzeugt wurden, wobei die Methode das Umwandeln der Genexpressionsdaten sowie folgende Schritte umfasst:

Konstruieren eines primären Modells für das Umwandeln von Genexpressionsdaten von einer ersten Profilierungsplattform zu einer zweiten Profilierungsplattform mithilfe von Probenexpressionsdaten, wobei das Konstruieren des primären Modells folgende Schritte umfasst:

Ermitteln mindestens einer gemeinsamen Expression zwischen einem ersten Satz von Nukleinsäureexpressionsdaten, die mithilfe einer ersten Profilierungsplattform abgeleitet wurden, und einem zweiten Satz von Nukleinsäureexpressionsdaten, die mithilfe einer zweiten Profilierungsplattform abgeleitet wurden, wobei die einzelnen gemeinsamen Expressionen jeweils einer Probe zugeordnet sind, die sowohl im ersten Satz als auch im zweiten Satz vorhanden ist; Durchführen einer Regressionsanalyse für die mindestens eine gemeinsame Expression, um jeweils einen Satz von Regressionsparametern für die einzelnen Proben zu erhalten; Auswählen mindestens eines Kandidatenmerkmals von der ersten Profilierungsplattform, das den mindestens einen Satz von Regressionsparametern vorhersagt; und Ermitteln eines primären Modells, das den einzelnen der mindestens einen ausgewählten Kandidatenmerkmale zugeordnet ist, wobei das primäre Modell die Daten der einzelnen Proben umwandelt, um die Genexpressionsdaten der ersten Profilierungsplattform in umgewandelte Daten umzuwandeln, die eine Verteilung aufweisen, die derjenigen der zweiten Profilierungsplattform ähnelt.

2. Die Methode gemäß Anspruch 1, die zudem das Erzeugen mindestens eines Satzes von Expressionsdaten mithilfe einer Profilierungsplattform umfasst, wobei es sich beim mindestens einen Satz von Expressionsdaten jeweils mindestens um den ersten und dem zweiten Satz von Expressionsdaten handelt.

3. Die Methode gemäß Anspruch 1, die zudem folgende Schritte umfasst:

Umwandeln der Probenexpressionsdaten mithilfe des konstruierten primären Modells; und Konstruieren eines kategorialen Modells mithilfe einer Regressionsanalyse, die jeweils mindestens Folgendes umfasst: (a) mindestens einige der umgewandelten Probenexpressionsdaten und (b) mindestens einige der gemeinsamen Ausdrücke.

4. Die Methode gemäß Anspruch 3, wobei jeweils mindestens: (a) das Auswählen mindestens einiger der umgewandelten Probenexpressionsdaten und (b) das Auswählen mindestens einiger der gemeinsamen Ausdrücke auf phänotypischen Daten oder einem beliebigen Faktor beruht, von dem bekannt ist, dass er eine plattformübergreifende Verzerrung hervorruft.

5. Die Methode gemäß Anspruch 3, die zudem das Wiederholen von Anspruch 4 mithilfe des kategorialen Modells umfasst, das aus den umgewandelten Probenexpressionsdaten konstruiert wurde, um die umgewandelten Probenexpressionsdaten umzuwandeln und daraus ein weiteres kategoriales Modell zu konstruieren.

6. Die Methode gemäß Anspruch 5, die zudem das Umwandeln eines Satzes von Expressionsdaten von der ersten Profilierungsplattform zur zweiten Profilierungsplattform umfasst, indem die konstruierten kategorischen Modelle in der Reihenfolge ihrer Konstruktion angewandt werden.

7. Die Methode gemäß Anspruch 1, wobei die erste Profilierungsplattform oder die zweite Profilierungsplattform aus der folgenden Gruppe ausgewählt wird: Agilent-Genexpressions-Mikroarrays, Affymetrix-Genprofilierungs-Array cGMP U133 P2/menschliches Genom U133 Plus 2.0/U133A 2.0, Illumina-Genom-Analyseeinheit/MiSeq/Next-Seq/HiSeq, NanoString nCounter SPRINT/MAX/FLEX und Oxford Nanopore MinION/PromethION/GridION.

8. Die Methode gemäß Anspruch 1, wobei die mindestens eine gemeinsame Expression anhand mindestens eines der folgenden übereinstimmenden Aspekte ermittelt wird: Positionen, Exone, Isoformen und Transkripte.

9. Die Methode gemäß Anspruch 1, wobei das mindestens eine Kandidatenmerkmal aus folgender Gruppe ausgewählt wird: mittlere Transkript-Expression, mittlere normalisierte Sondenintensität, Anzahl der erkannten Gene, Anzahl der Auslesungen pro Probe, durchschnittliche Anzahl der Auslesungen pro Exon/Gen/Isoform, Leseabdeckung und Probenstatistik.

10. Die Methode gemäß Anspruch 5, wobei die einzelnen Modelle aus folgender Gruppe ausgewählt werden: lineares Modell, logarithmisches Modell, stückweise lineares Modell und Regressionsmodell.

11. Ein Gerät für das direkte Kombinieren der von heterogenen Plattformen erzeugten Genexpressionsdaten durch Umwandeln der Genexpressionsdaten, um eine computergestützte Analyse durchzuführen, wobei das Gerät Folgendes umfasst:

einen Prozessor;
eine Schnittstelle; und
von einem Computer ausführbare Anweisungen, die auf dem Prozessor umgesetzt werden, um folgende Schritte durchzuführen:

Konstruieren eines primären Modells für das Umwandeln von Genexpressionsdaten von einer ersten Profilierungsplattform zu einer zweiten Profilierungsplattform mithilfe von Probenexpressionsdaten, sodass die Gesamtverteilung der umgewandelten Daten derjenigen der zweiten Plattform ähnelt,
wobei die auf einem Computer ausführbaren Anweisungen für das Konstruieren des primären Modells das Berechnen von ausführbaren Anweisungen für folgende Schritte umfasst:

Ermitteln mindestens einer gemeinsamen Expression zwischen einem ersten Satz von Nukleinsäureexpressionsdaten, die mithilfe einer ersten Profilierungsplattform abgeleitet wurden, und einem zweiten Satz von Nukleinsäureexpressionsdaten, die mithilfe einer zweiten Profilierungsplattform abgeleitet wurden, wobei die einzelnen gemeinsamen Expressionen jeweils einer Probe zugeordnet sind, die sowohl im ersten Satz als auch im zweiten Satz vorhanden ist;
Durchführen einer Regressionsanalyse für die mindestens eine gemeinsame Expression, um jeweils einen Satz von Regressionsparametern für die einzelnen Proben zu erhalten;
Auswählen mindestens eines Kandidatenmerkmals von der ersten Profilierungsplattform, das den mindestens einen Satz von Regressionsparametern vorhersagt; und
Ermitteln eines primären Modells, das den einzelnen der mindestens einen ausgewählten Kandidatenmerkmale zugeordnet ist, wobei das primäre Modell die Daten der einzelnen Proben umwandelt, um die Genexpressionsdaten der ersten Profilierungsplattform in umgewandelte Daten umzuwandeln, die eine Verteilung aufweisen, die derjenigen der zweiten Profilierungsplattform ähnelt.

12. Das Gerät gemäß Anspruch 11, wobei die Schnittstelle zudem mindestens einen Satz von Expressionsdaten von einer Profilierungsplattform abruft, wobei es sich beim mindestens einen Satz von Expressionsdaten jeweils mindestens um den ersten und dem zweiten Satz von Expressionsdaten handelt.

**13.** Das Gerät gemäß Anspruch 11, das zudem von einem Computer ausführbare Anweisungen umfasst, die auf dem Prozessor umgesetzt werden, um folgende Schritte durchzuführen:

Umwandeln der Probenexpressionsdaten mithilfe des konstruierten primären Modells; und
Konstruieren eines kategorialen Modells mithilfe einer Regressionsanalyse, die jeweils mindestens Folgendes umfasst: (a) mindestens einige der umgewandelten Probenexpressionsdaten und (b) mindestens einige der gemeinsamen Ausdrücke.

**14.** Das Gerät gemäß Anspruch 13, wobei jeweils mindestens: (a) das Auswählen mindestens einiger der umgewandelten Probenexpressionsdaten und (b) das Auswählen mindestens einiger der gemeinsamen Ausdrücke auf phänotypischen Daten oder einem beliebigen Faktor beruht, von dem bekannt ist, dass er eine plattformübergreifende Verzerrung hervorruft.

**15.** Das Gerät gemäß Anspruch 13, das zudem auf einem Computer ausführbare und auf dem Prozessor umgesetzte Anweisungen zum Wiederholen von Anspruch 13 mithilfe des kategorialen Modells umfasst, das aus den umgewandelten Probenexpressionsdaten konstruiert wurde, um die umgewandelten Probenexpressionsdaten umzuwandeln und daraus ein weiteres kategoriales Modell zu konstruieren.

**16.** Das Gerät gemäß Anspruch 15, das zudem auf einem Computer ausführbare und auf dem Prozessor umgesetzte Anweisungen zum Umwandeln eines Satzes von Expressionsdaten von der ersten Profilierungsplattform zur zweiten Profilierungsplattform umfasst, indem die konstruierten kategorischen Modelle in der Reihenfolge ihrer Konstruktion angewandt werden.

**17.** Das Gerät gemäß Anspruch 11, wobei die erste Profilierungsplattform oder die zweite Profilierungsplattform aus der folgenden Gruppe ausgewählt wird: Agilent-Genexpressions-Mikroarrays, Affymetrix-Genprofilierungs-Array cGMP U133 P2/menschliches Genom U133 Plus 2.0/U133A 2.0, Illumina-Genom-Analyseeinheit/MiSeq/NextSeq/HiSeq, NanoString nCounter SPRINT/MAX/FLEX und Oxford Nanopore MinION/PromethION/GridION.

**18.** Das Gerät gemäß Anspruch 11, wobei die auf einem Computer ausführbaren Anweisungen zum Ermitteln mindestens einer gemeinsamen Expression von einem Computer ausführbare Anweisungen zum Ermitteln mindestens einer gemeinsamen Expression anhand mindestens eines der folgenden übereinstimmenden Aspekte umfasst: Positionen, Exone, Isoformen und Transkripte.

**19.** Das Gerät gemäß Anspruch 11, wobei das mindestens eine Kandidatenmerkmal aus folgender Gruppe ausgewählt wird: mittlere Transkript-Expression, mittlere normalisierte Sondenintensität, Anzahl der erkannten Gene, Anzahl der Auslesungen pro Probe, durchschnittliche Anzahl der Auslesungen pro Exon/Gen/Isoform, Leseabdeckung und Probenstatistik.

**20.** Das Gerät gemäß Anspruch 15, wobei die einzelnen Modelle aus folgender Gruppe ausgewählt werden: lineares Modell, logarithmisches Modell, stückweise lineares Modell und Regressionsmodell.

**Revendications**

**1.** Procédé mis en œuvre par ordinateur permettant de faciliter la combinaison directe des données d'expression génique générées par des plates-formes hétérogènes, ledit procédé comprenant la transformation de données d'expression génique, ledit procédé comprenant :

la construction d'un modèle primaire pour transformer des données d'expression génique d'une première plateforme de profilage en celles d'une seconde plateforme de profilage à l'aide des données d'expression d'échantillon,
dans lequel la construction du modèle primaire comprend :

l'identification d'au moins une expression commune entre un premier ensemble de données d'expression d'acide nucléique dérivées à l'aide d'une première plateforme de profilage et un second ensemble de données d'expression d'acide nucléique dérivées à l'aide d'une seconde plateforme de profilage, chaque expression commune étant associée à un échantillon présent à la fois dans le premier ensemble et second ensemble ;

la réalisation d'une analyse de régression sur l'au moins une expression commune, résultant en un ensemble de paramètres de régression pour chaque échantillon ;

la sélection d'au moins une caractéristique candidate à partir de la première plateforme de profilage, laquelle prédit l'au moins un ensemble de paramètres de régression ; et

l'identification d'un modèle primaire associé à chacune de l'au moins une caractéristique candidate sélectionnée, le modèle primaire étant configuré pour une transformation de données par échantillon pour transformer les données d'expression génique de la première plateforme de profilage en données transformées présentant une distribution, laquelle ressemble à celle de la seconde plateforme de profilage.

2. Procédé selon la revendication 1, comprenant en outre la génération d'au moins un ensemble de données d'expression à l'aide d'une plateforme de profilage, l'au moins un ensemble de données d'expression étant le premier et/ou le second ensemble de données d'expression.

3. Procédé selon la revendication 1, comprenant en outre :

la transformation des données d'expressions d'échantillon à l'aide du modèle primaire construit ; et
la construction d'un modèle catégoriel par analyse de régression à partir des éléments suivants : (a) au moins certaines des données d'expressions d'échantillons transformées et/ou (b) au moins certaines des expressions communes.

4. Procédé selon la revendication 3, dans lequel : (a) la sélection des au moins certaines des données d'expression d'échantillon transformées et/ou (b) la sélection des au moins certaines des expressions communes sont basées sur des données phénotypiques ou sur tout facteur connu pour introduire un biais multiplateforme.

5. Procédé selon la revendication 3, comprenant en outre l'itération de la revendication 4 à l'aide du modèle catégoriel construit à partir des données d'expression d'échantillon transformées pour transformer les données d'expression d'échantillon transformées et la construction d'un autre modèle catégoriel à partir de celles-ci.

6. Procédé selon la revendication 5, comprenant en outre la transformation d'un ensemble de données d'expression de la première plateforme de profilage en la seconde plateforme de profilage par application des modèles catégoriels construits dans l'ordre de leur construction.

7. Procédé selon la revendication 1, dans lequel la première plateforme de profilage ou la seconde plateforme de profilage est sélectionnée dans le groupe constitué par des puces à expression génique d'Agilent, une puce à profil génique cGMP U133 P2 / génome humain U133 Plus 2.0 /U133A 2.0 d'Affymetrix, un analyseur de génomes /MiSeq/NextSeq/HiSeq d'Illumina, un nCounter SPRINT/MAX/FLEX de NanoString, et MinION/PromethION/GridION d'Oxford Nanopore.

8. Procédé selon la revendication 1, dans lequel l'au moins une expression commune est identifiée par des positions génomiques correspondantes et/ou des exons correspondants et/ou des isoformes correspondantes et/ou des transcriptions correspondantes.

9. Procédé selon la revendication 1, dans lequel l'au moins une caractéristique candidate est choisie dans le groupe constitué par l'expression moyenne du transcrit, l'intensité moyenne normalisée de la sonde, le nombre de gènes détectés, le nombre de lectures par échantillon, le nombre moyen de lectures par exon/gène/isoforme, le champ d'application de lecture et des statistiques d'échantillon.

10. Procédé selon la revendication 5, dans lequel chacun des modèles est sélectionné dans le groupe constitué par un modèle linéaire, un modèle logarithmique, un modèle linéaire par morceaux et un modèle de régression.

11. Appareil de facilitation de la combinaison directe, pour une analyse informatique, des données d'expression génique générées par des plates-formes hétérogènes par transformation des données d'expression génique, ledit appareil comprenant :

un processeur ;
une interface ; et
des instructions exécutables par ordinateur opérant sur ledit processeur :

pour construire un modèle primaire pour transformer les données d'expression génique d'une première plateforme de profilage en celles d'une seconde plateforme de profilage à l'aide des données d'expression d'échantillon de telle sorte que la distribution globale des données transformées ressemble à celle de la seconde plateforme,

dans lequel les instructions exécutables par ordinateur destinées à la construction du modèle primaire comprennent le calcul des instructions exécutables :

pour identifier au moins une expression commune entre un premier ensemble de données d'expression d'acide nucléique dérivées à l'aide d'une première plateforme de profilage et un second ensemble de données d'expression d'acide nucléique dérivées à l'aide d'une seconde plateforme de profilage, chaque expression commune étant associée à un échantillon présent à la fois dans le premier ensemble et second ensemble ;

pour réaliser d'une analyse de régression sur l'au moins une expression commune, résultant en un ensemble de paramètres de régression pour chaque échantillon ;

pour sélectionner au moins une caractéristique candidate à partir de la première plateforme de profilage, laquelle prédit l'au moins un ensemble de paramètres de régression ; et

pour identifier un modèle primaire associé à chacune des au moins une caractéristique candidate sélectionnée le modèle primaire étant configuré pour une transformation de données par échantillon pour transformer les données d'expression génique de la première plateforme de profilage en données transformées présentant une distribution, laquelle ressemble à celle de la seconde plateforme de profilage.

12. Appareil selon la revendication 11, dans lequel l'interface est configurée pour recevoir au moins un ensemble de données d'expression d'une plateforme de profilage, l'au moins un ensemble de données d'expression étant le premier et/ou le second ensemble de données d'expression.

13. Appareil selon la revendication 11, comprenant en outre des instructions exécutables par ordinateur opérant sur ledit processeur :

pour transformer les données d'expressions d'échantillon à l'aide du modèle primaire construit ; et
pour construire un modèle catégoriel par analyse de régression à partir des éléments suivants : (a) au moins certaines des données d'expressions d'échantillons transformées et/ou (b) au moins certaines des expressions communes.

14. Appareil selon la revendication 13, dans lequel : (a) la sélection des au moins certaines des données d'expression d'échantillon transformées et/ou (b) la sélection des au moins certaines des expressions communes sont basées sur des données phénotypiques ou sur tout facteur connu pour introduire un biais multiplateforme.

15. Appareil selon la revendication 13, comprenant en outre des instructions exécutables par ordinateur fonctionnant sur ledit processeur destinées à l'itération de la revendication 13 à l'aide du modèle catégoriel construit à partir des données d'expression d'échantillon transformées pour transformer les données d'expression d'échantillon transformées et pour construire un autre modèle catégoriel à partir de celles-ci.

16. Appareil selon la revendication 15, comprenant en outre des instructions exécutables par ordinateur opérant sur ledit processeur destinées à la transformation d'un ensemble de données d'expression de la première plateforme de profilage en celles de la seconde plateforme de profilage par application des modèles catégoriels construits dans l'ordre de leur construction.

17. Appareil selon la revendication 11, dans lequel la première plateforme de profilage ou la seconde plateforme de profilage est sélectionnée dans le groupe constitué par des puces à expression génique d'Agilent, une puce à profil génique cGMP U133 P2 / génome humain U133 Plus 2.0 /U133A 2.0 d'Affymetrix, un analyseur de génomes /MiSeq/NextSeq/HiSeq d'Illumina, un nCounter SPRINT/MAX/FLEX de NanoString, et MinION/PromethION/GridION d'Oxford Nanopore.

18. Appareil selon la revendication 11, dans lequel les instructions exécutables par ordinateur destinées à l'identification d'au moins une expression commune comprennent des instructions exécutables par ordinateur pour identifier l'au moins une expression commune par des positions génomiques correspondantes et/ou des exons correspondants et/ou des isoformes correspondantes et/ou des transcriptions correspondantes.

**19.** Appareil selon la revendication 11, dans lequel l'au moins une caractéristique candidate est choisie dans le groupe constitué par l'expression moyenne du transcrit, l'intensité moyenne normalisée de la sonde, le nombre de gènes détectés, le nombre de lectures par échantillon, le nombre moyen de lectures par exon/gène/isoforme, le champ d'application de lecture et des statistiques d'échantillon.

**20.** Appareil selon la revendication 15, dans lequel chacun des modèles est sélectionné dans le groupe constitué par un modèle logarithmique, un modèle linéaire, un modèle linéaire par morceaux et un modèle de régression.

**FIG. 1**

*FIG. 2*

```
┌─────────────────────────┐
│   IDENTIFY COMMON        │
│   EXPRESSIONS            │
│   200                    │
└─────────────────────────┘
             │
             ▼
┌─────────────────────────┐
│   PERFORM REGRESSION     │
│   ON COMMON              │
│   EXPRESSIONS            │
│   204                    │
└─────────────────────────┘
             │
             ▼
┌─────────────────────────┐
│   IDENTIFY CANDIDATE     │
│   FEATURE(S) FOR         │
│   REGRESSION VALUES      │
│   208                    │
└─────────────────────────┘
             │
             ▼
┌─────────────────────────┐
│   SELECT PREDICTIVE      │
│   FEATURES               │
│   212                    │
└─────────────────────────┘
             │
             ▼
┌─────────────────────────┐
│   PROVIDE PRIMARY        │
│   MODEL                  │
│   216                    │
└─────────────────────────┘
```

*FIG. 3*

SUBSTITUTE
PREDICTIVE FEATURES
INTO MODELS
300

INSERT REGRESSION
PARAMS INTO
REGRESSION MODEL
304

ESTIMATE EXPRESSION
PROFILE
308

**FIG. 4**

RECEIVE TRANSFORMED DATA
400

REGRESS ALL SAMPLE DATA FOR EACH CATEGORY
404

RETAIN MODELS THAT IMPROVE ACCURACY
408

IDENTIFY REGRESSION PARAMETERS
412

*FIG. 5*

```
INSERT REGRESSION
   PARAMS INTO
REGRESSION MODEL
       500
```

```
ESTIMATE EXPRESSION
      PROFILE
        504
```

# FIG. 6

**FIG. 7**

(A) Linear Model for Slope 1

(B) Linear Model for Intercept 1

(C) Linear Model for Slope 2

(D) Linear Model for Intercept 2

# FIG. 8

RNASeq vs. MA Expression for OV Sample TCGA-04-1348-01A

(A)

RNASeq vs. MA Expression for OV Sample TCGA-04-1362-01A

(B)

FIG. 9

(A)

(B)

EP 3 207 133 B1

# *FIG. 10*

1000

Sample Data
1004

| RECEIVER 1002 | → | MEMORY 1006 | → | PROCESSOR 1008 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62065367 B

### Non-patent literature cited in the description

- **BAUER MICHAEL A et al.** Leveraging the new with the old: providing a framework for the integration of historic microarray studies with next generation sequencing. *BMC BIOINFORMATICS,* 01 October 2014, vol. 15 (11

- **SHWETA S CHAVAN et al.** Towards the integration, annotation and association of historical microarray experiments with RNA-seq. *BMC BIOINFORMATICS, BIOMED CENTRAL,* 09 October 2013, vol. 14 (14), ISSN 1471-2105, 1-11